# EUROPÄISCHE PATENTSCHRIFT

(11) **EP 0 697 853 B1**
(45) Veröffentlichungstag und Bekanntmachung des Hinweises auf die Patenterteilung: **23.06.1999**
(21) Anmeldenummer: 95907604.3
(22) Anmeldetag: 25.01.1995
(51) Int. Cl.: A61K 7/06, A61K 7/50

(54) **HAARPFLEGEMITTEL**
HAIR CARE AGENT
PRODUIT D'HYGIENE CAPILLAIRE

(30) Priorität: 15.03.1994 DE 4408727
(43) Veröffentlichungstag der Anmeldung: 28.02.1996
(73) Patentinhaber: Wella Aktiengesellschaft, 64274 Darmstadt (DE)
(72) Erfinder: KELLER, Walter, D-64372 Ober-Ramstadt (DE); KISCHKA, Karl-Heinz, D-64293 Darmstadt (DE)
(86) Internationale Anmeldenummer: EP9500259
(87) Internationale Veröffentlichungsnummer: WO9524882

(56) Entgegenhaltungen:
- WO-A-91/15186
- DE-C- 3 719 086
- US-A- 4 983 418
- SOAP, COSMETICS, CHEMICAL SPECIALTIES., Bd.62, Nr.4, April 1986, NEW YORK, USA Seiten 34 - 39 M. S. STARCH 'Silicones for Conditioning Damaged Hair'

## Beschreibung

Gegenstand der Erfindung ist ein Haarpflegemittel auf wäßriger oder wäßrig-alkoholischer Basis, welches eine Kombination aus einem Gemisch aus cyclischem Dimethylpolysiloxan und alpha-Hydroxy,omega-hydroxypoly[oxy(dimethylsilylen)] mit einem kationischen Tensid, einem diquaternären Polydimethylsiloxan, einem kationischen Polymer und Polyvinylpyrrolidon und/oder Polyvinylpyrrolidon/Vinylacetat-Copolymer enthält, das nicht ausgespült wird, sondern im Haar verbleiben kann.

Die Haare werden durch Einwirkungen verschiedener Art in ihren physikalischen, chemischen und morphologischen Eigenschaften negativ beeinflußt. So wird das Haar durch kosmetische Behandlungen, wiederholtes Bleichen, Dauerwellen und Färben, aber auch schon häufiges Waschen der Haare mit enfettenden Tensiden, durch Klimaeinflüsse, wie Luftfeuchtigkeits- und Temperaturunterschiede oder durch intensive Einwirkung von Sonnenlicht, sowie durch mechanische Behandlung, wie Bürsten, Kämmen, Frottieren, besonders im Bereich der Haarspitzen stark strapaziert und geschädigt. Das Haar wird spröde und verliert seinen Glanz. Geschädigtes Haar lädt sich beim Bürsten und Kämmen elektrostatisch auf. Die Haaroberfläche wird aufgerauht und es kommt zu Verfilzungen und Verknotungen. Die Folge ist eine außerordentlich schlechte Kämmbarkeit.

Haarpflegemittel mit einer kämmbarkeitsverbesserenden und pflegenden Wirkung, welche eine Verbesserung des Haarzustandes bewirken, haben daher eine erhebliche Bedeutung erlangt. Derartige Mittel liegen üblicherweise in Form von Emulsionen oder Suspensionen vor, welche Fettalkohole, Wachse, Öle sowie anionische, kationische oder nichtionische Emulgatoren enthalten.

Haarpfegemittel auf der Basis der organischen Polysiloxane werden in der WO-A-91/15186 beschrieben.

Derartige Haarpflegemittel haben den Nachteil, daß sie nach der Einwirkungszeit mit Wasser wieder aus dem Haar gespült werden müssen, da es sonst zu einer unerwünschten Belastung der Haare im trockenen Zustand kommt. Die Haare werden glanzlos, verlieren an Elastizität und wirken ungepflegt.

Es bestand daher die Aufgabe ein Haarpflegemittel zur Verfügung zu stellen, das nach der Einwirkungszeit nicht wieder ausgespült werden muß und alle Vorteile eines auszuspülenden Haarpflegemittels besitzt, die vorstehend geschilderten Nachteile der auszuspülenden Haarpflegemittel jedoch vermeidet.

Es wurde nunmehr gefunden, daß durch ein Haarpflegemittel, das nicht ausgespült werden muß, auf wäßriger oder wäßrig-alkoholischer Basis, dadurch gekennzeichnet, daß es
(A) 0,5 bis 2,5 Gewichtsprozent eines Gemisches aus 87 Gewichtsprozent cyclischem Dimethylpolysiloxan der allgemeinen Formel (I) 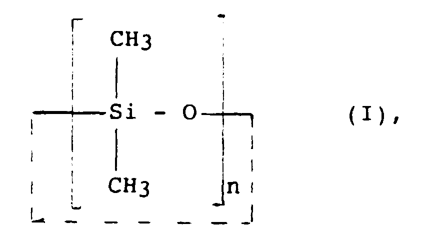 wobei n im Mittel 4 bis 6 bedeutet, und 13 Gewichtsprozent alpha-Hydroxy,omega-hydroxypoly[oxy(dimethylsilylen)],
(B) 0,25 bis 1,25 Gewichtsprozent eines diquaternären Polydimethylsiloxans,
(C) 0,10 bis 0,50 Gewichtsprozent mindestens eines kationischen Polymers, das kein Silikonpolymer ist,
(D) 0,05 bis 0,50 Gewichtsprozent mindestens eines kationischen Tensids und
(E) 0,10 bis 5,00 Gewichtsprozent Polyvinylpyrrolidon und/oder Polyvinylpyrrolidon/Vinylacetat-Copolymer
enthält und frei von anionischen Tensiden ist, die gestellte Aufgabe in hervorragender Weise erfüllt wird.

Das erfindungsgemäße Haarpflegemittel enthält die Komponente (A) bevorzugt in einer Menge von 0,50 bis 1,5 Gewichtsprozent. Ein geeignetes Gemisch aus 87 Gewichtsprozent cyclischem Dimethylpolysiloxan der allgemeinen Formel (I) (CTFA-Bezeichnung: Cyclomethicone, CTFA International Cosmetic Ingredient Dictionary, USA 1991) und 13 Gewichtsprozent alpha-Hydroxy,omega-hydroxypoly[oxy(dimethylsilylen)](CTFA-Bezeichnung: Dimethiconol) wird beispielsweise von der Firma Dow Corning GmbH, Wiesbaden, BRD unter dem Handelsnamen Dow Corning® Fluid Q2-1401 vertrieben.

Das erfindungsgemäße Haarpflegemittel enthält bevorzugt 0,25 bis 0,75 Gewichtsprozent der Komponente (B). Als Komponente (B) enthält das erfindungsgemäße Mittel bevorzugt diquaternäre Polydimethylsiloxane der allgemeinen Formel (II) 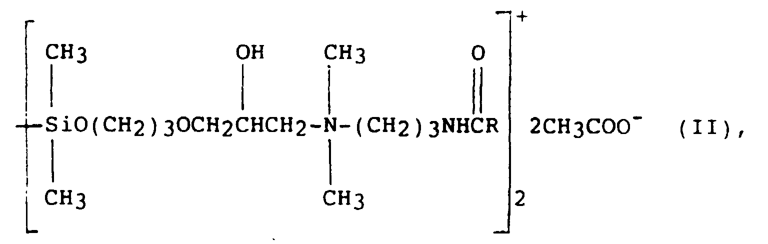 wobei R einen Kokosfettsäurealkylrest darstellt (CTFA-Bezeichnung: Quaternium 80).

Als Komponente (B) geeignete diquaternäre Polydimethylsiloxane der allgemeinen Formel (II) werden beispielsweise von der Firma TH. Goldschmidt AG, Essen, BRD unter den Bezeichnungen Abil® Quat 3270 und Abil® Quat 3272 vertrieben.

Das erfindungsgemäße Mittel enthält bevorzugt 0,10 bis 0,30 Gewichtsprozent der Komponente (C). Das kationische Polymer der Komponente (C) ist bevorzugt ausgewählt aus mit Dimethylsulfat quaternisiertem Vinylpyrrolidon/Dimethylaminoethylmethacrylat-Copolymeren, Diallyldimethylammoniumchlorid/Acrylamid-Copolymeren, Dimethyldiallylammoniumchlorid-Polymeren, polymeren quaternären Ammoniumsalzen von Hydroxyethylcellulose und Vinylimidazoliniummethochlorid/Vinylpyrrolidon-Copolymeren oder deren Gemisch.

Als Komponente (C) geeignete Diallyldimethylammoniumchlorid/Acrylamid-Copolymere werden beispielsweise von der Firma Calgon, Pittsburgh, USA in Form einer 8prozentigen wäßrigen Lösung unter der Handelsbezeichnung Merquat® 550, geeignete Dimethyldiallylammoniumchlorid-Polymere werden von der vorstehend genannten Firma in Form einer 40prozentigen wäßrigen Lösung unter der Handelsbezeichnung Merquat® 100 vertrieben. Als Komponente (C) geeignete polymere quaternäre Ammoniumsalze von Hydroxyethylcellulose werden beispielsweise von der Firma Union Carbide, Genf, Schweiz unter der Handelsbezeichnung Polymer JR® vertrieben.

Das erfindungsgemäße Mittel enthält als Komponente (C) besonders bevorzugt ein mit Dimethylsulfat quaternisiertes Vinylpyrrolidon/Dimethylaminoethylmethacrylat-Copolymer, das beispielsweise von der Firma Gaf Co., New York, USA in Form einer 20prozentigen wäßrigen Lösung unter der Handelsbezeichnung Gafquat® 755 N vertrieben wird, oder ein Vinylimidazoliniummethochlorid/Vinylpyrrolidon-Copolymer, das beispielsweise von der Firma BASF AG, Ludwigshafen, BRD in Form einer 40prozentigen wäßrigen Lösung unter der Handelsbezeichnung Luviquat® FC 905 vertrieben wird, oder deren Gemisch.

Das erfindungsgemäße Haarpflegemittel enthält bevorzugt 0,10 bis 0,30 Gewichtsprozent der Komponente (D).

Beispiele für als Komponente (D) des erfindungsgemäßen Mittels geeignete kationische Tenside sind die Chloride oder Bromide von Alkyldimethylbenzylammoniumsalzen, Alkyltrimethylammoniumsalze, beispielsweise Cetyltrimethylammoniumchlorid oder -bromid, Tetradecyltrimethylammoniumchlorid oder -bromid, Alkyldimethylhydroxyethylammoniumchloride oder -bromide, die Dialkyldimethylammoniumchloride oder -bromide, Alkylpyridiniumsalze, beispielsweise Lauryl- oder Cetylpyridiniumchlorid, Alkylamidethyltrimethylammoniumethersulfate sowie Verbindungen mit kationischen Charakter wie Aminoxide, beispielsweise Alkylmethylaminoxide oder Alkylaminoethyldimethylammioxide.

Das erfindungsgemäße Mittel enthält als Komponente (D) bevorzugt Cetyltrimethylammoniumchlorid das beispielsweise in Form einer 26prozentigen wäßrigen Lösung unter der Handelsbezeichnung Dehyquart® A von der Firma Henkel KGaA, Düsseldorf, BRD und in Form einer 29prozentigen wäßrigen Lösung unter der Handelsbezeichnung Genamin® CTAC von der Firma Hoechst AG, Frankfurt, BRD sowie in Form einer 50prozentigen Lösung in Isopropanol unter der Handelsbezeichnung Arquad® 16-50 von der Firma Akzo Chemicals GmbH, Düren, BRD vertrieben wird.

Das erfindungsgemäße Mittel enthält die Komponente (E) bevorzugt in einer Menge von 0,2 bis 3,0 Gewichtsprozent. Geeignete Polyvinylpyrrolidone werden beispielsweise unter der Handelsbezeichnung Luviskol® K 90 von der Firma BASF AG, Ludwigshafen, BRD und unter der Handelsbezeichnung PVP/K® 90 von der Firma ISP, Surrey, Großbritannien vertrieben. Von den als Komponente (E) des erfindungsgemäßen Mittels geeigneten Polyvinylpyrrolidonen ist jenes bevorzugt, das ein mittleres Molekulargewicht von 700.000 aufweist und beispielsweise von der Firma BASF AG, Ludwigshafen, BRD unter der Handelsbezeichnung Luviskol® K 90 vertrieben wird.

Geeignete Polyvinylpyrrolidon-Vinylacetat-Copolymere werden beispielsweise unter der Handelsbezeichnung Luviskol®-VA von der Firma BASF AG, Ludwigshafen, BRD vertrieben. Von den als Komponente (E) des erfindungsgemäßen Mittels geeigneten Polyvinylpyrrolidon/Vinylacetat-Copolymeren sind jene bevorzugt, die ein Verhältnis der Polyvinylpyrrolidonmonomere zu den Vinylacetatmonomeren von 60 zu 40 oder von 30 zu 70 aufweisen und beispielsweise von der Firma BASF AG, Ludwigshafen, BRD unter der Handelsbezeichnung Luviskol® VA 64 beziehungsweise Luviskol® VA 37 vertrieben werden.

Das erfindungsgemäße Haarpflegemittel kann für Haarpflegemittel geeignete amphotere und nicht-ionische Tenside in einer Menge von 0,1 bis 5 Gewichtsprozent enthalten.

Von den für Haarpflegemittel geeigneten nicht-ionischen Tensiden seien beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Tetradecyl-, Cetyl-, Oleyl- und Stearylalkohol, allein oder im Gemisch; die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül; Fettsäurealkanolamide, oxethylierte Sorbitanfettsäureester, Alkylpolyglycoside und oxethyliertes hydriertes Rizinusöl genannt.

Als Alkylpolyglycosid ist beispielsweise Laurylpolyglucose geeignet, die in Form einer 30prozentigen wäßrigen Lösung unter der Handelsbezeichnung Oramix® NS 30 von der Firma Seppic Inc., New Jersey, USA vertrieben wird.

Als oxethyliertes hydriertes Rizinusöl ist beispielsweise mit 45 Ethylenoxideinheiten ethoxyliertes hydriertes Rizinusöl geeignet, das unter der Handelsbezeichnung Cremophor® RH 410 von der BASF AG, Ludwigshafen, BRD vertrieben wird.

Von den für Haarpflegemittel geeigneten amphoteren Tensiden seien beispielsweise die Carboxylderivate von Imidazol, die N-Alkylbetaine, die N-Alkylaminobetaine, die N-Alkylsulfobetaine, die N-Alkylaminopropionate, die Alkyldimethylammoniumacetate, die C₁₂- bis C₁₈-Alkyldimethylcarboxymethylammoniumsalze sowie die Fettsäurealkylamidobetaine, beispielsweise Kokosfettsäureamidopropylbetain, genannt.

Das Haarpflegemittel gemäß der vorliegenden Erfindung kann zusätzlich alle diejenigen Bestandteile enthalten, die in Haarpflegemitteln üblicherweise eingesetzt werden, insbesondere Schaumsynergisten, Schaumstabilisatoren Sequestriermittel, Naturstoffe, Pigmente, Parfümöle in einer Menge von 0,5 bis 5,0 Gewichtsprozent; Trübungsmittel, wie zum Beispiel Ethylenglykoldistearat, in einer Menge von etwa 0,5 bis 5,0 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Verdickungsmittel, wie beispielsweise Kokosfettsäurediethanolamid oder Hydroxyalkylcellulose, in einer Menge von 0,5 bis 10,0 Gewichtsprozent; Puffersubstanzen, wie beispielsweise Natriumcitrat oder Natriumphosphat, in einer Menge von 0,1 bis 1,0 Gewichtsprozent; sowie Farbstoffe zum Anfärben des Mittels, wie zum Beispiel Fluorescein-Natriumsalz, Gelb ZN3 (C.I. 47 005), in einer Menge von 0,1 bis 0,5 Gewichtsprozent; weiterhin haarpflegende Zusätze, wie zum Beispiel Fettsäureester, Fettalkohole, Fettsäureglyceride, natürliche, modifizierte natürliche oder synthetische Polymere, wie zum Beispiel Schellack, anionische oder nichtionische Cellulosederivate, Chitosan, Chitin- oder Chitosanderivate oder Polymerisate von Acrylsäurederivaten; Pflegestoffe, wie zum Beispiel Lanolinderivate, Cholesterin und Pantothensäure, in einer Menge von 0,1 bis 10 Gewichtsprozent; außerdem physiologisch verträgliche anorganische Salze, wie zum Beispiel Natriumchlorid und Natriumsulfat; sowie ferner Feuchthaltemittel; Lichtschutzmittel; Antioxidantien; Komplexbildner; Antischuppenwirkstoffe; kosmetische Öle und Wachse sowie Konservierungsstoffe; soweit solche Zusätze nützlich und zweckmäßig erscheinen und mit den Bestandteilen des erfindungsgemäßen Mittels verträglich sind.

Das erfindungsgemäße Mittel weist bevorzugt einen Wassergehalt von 95 bis 99 Gewichtsprozent auf und kann Alkohol in einer Menge von 0,5 bis 20 Gewichtsprozent enthalten. Als Alkohol kommen hierbei insbesondere die für kosmetische Zwecke üblicherweise verwendeten niederen Alkohole mit 1 bis 4 Kohlenstoffatomen, wie zum Beispiel Ethanol und Isopropanol, in Betracht.

Das erfindungsgemäße Mittel weist einen pH-Wert von 2 bis 10, bevorzugt von 3 bis 8, auf.

In einer besonderen Ausführungsform des erfindungsgemäßen Haarpflegemittels enthält dieses zur gleichzeitigen Tönung des Haares 0,001 bis 2,0 Gewichtsprozent mindestens eines direkt auf das Haar aufziehenden Haarfarbstoffs, der beispielsweise aus den folgenden Klassen direkt auf das Haar aufziehender Haarfarbstoffe ausgewählt sein kann: aromatische Nitrofarbstoffe, zum Beispiel 1,4-Diamino-2-nitrobenzol, Azofarbstoffe, zum Beispiel Acid Brown 4 (C.I. 14 805), Anthrachinonfarbstoffe, zum Beispiel Disperse Violet 4 (C.I. 61 105) und Triphenylmethanfarbstoffe, zum Beispiel Basic Violet 14 (C.I. 42 510), wobei die Farbstoffe je nach Art ihrer Substituenten sauren, nichtionogenen oder basischen Charakter haben können.

Das erfindungsgemäße Mittel liegt bevorzugt in Form einer wäßrigen oder wäßrig-alkoholischen Dispersion, oder einer sogenannten Schütteldispersion, bei der zwei Phasen durch Schütteln für die Applikationszeit in eine Dispersion überführt werden, vor und kann unter Verwendung eines Treibmittels oder mit Hilfe einer mechanisch betriebenen Sprühvorrichtung oder Schaumerzeugungsvorrichtung versprüht oder als Schaum abgegeben werden. Das erfindungsgemäße Mittel wird bevorzugt unter Verwendung eines Treibmittels oder mit Hilfe einer mechanisch betriebenen Schaumerzeugungsvorrichtung als Schaum abgegeben.

Wenn das erfindungsgemäße Mittel mit Hilfe eines Treibmittels versprüht oder als Schaum abgegeben wird, so enthält es bevorzugt 3 bis 20 Gewichtsprozent des Treibmittels und wird in einen Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise N₂, N₂O und CO₂, sowie Gemische der vorstehend genannten Treibmittel geeignet.

Unter mechanischen Sprühvorrichtungen oder Schaumerzeugungsvorrichtungen sind solche Vorrichtungen zu verstehen, welche das Versprühen oder Aufschäumen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Als geeignete mechanische Schaumerzeugungsvorrichtung kann beispielsweise der in der EP-B 0 460 154 beschriebene Aufsatz mit einer Schaumerzeugungseinrichtung auf einem flexiblen Behälter verwendet werden.

Das erfindungsgemäße Haarpflegemittel wird, üblicherweise nach der Haarwäsche, in dem handtuchtrockenen Haar je nach Haarfülle in einer Menge von 10 bis 30 Gramm verteilt. Anschließend wird das Haar, ohne das erfindungsgemäße Mittel zuvor auszuspülen, durchgekämmt, gegebenenfalls zur Frisur geformt und getrocknet.

Es ist ein besonderer Vorzug des erfindungsgemäßen Mittels, daß es ohne auszuspülen im Haar verbleiben kann. Der Anwender erspart sich dadurch die Zeit und Mühe des Ausspülens.

Die mit dem erfindungsgemäßen Haarpflegemittel behandelten Haare weisen eine hervorragende Naß- und Trockenkämmbarkeit und einen angenehmen Griff auf. Das Haar hat einen ansprechenden Glanz und zeichnet sich durch Spannkraft und Volumen aus.

Folgende Beispiele sollen den Gegenstand der Erfindung näher erläutern.

### Beispiele

| **Beispiel 1:** | **Haarpflegeschaum** |
|---|---|
| 0,90 g | eines Gemisches aus 87 Gewichtsprozent cyclischem Dimethylpolysiloxan und 13 Gewichtsprozent alpha-Hydroxy, omega-hydroxypoly[oxy(dimethylsilylen)] (Dow Corning® Fluid Q2-1401 der Firma Dow Corning GmbH, BRD) |
| 0,90 g | diquaternäres Polydimethylsiloxan (Abil®-Quat 3272 der Fa. TH. Goldschmidt AG, BRD) |
| 0,18 g | mit Dimethylsulfat quaternisiertes Vinylpyrrolidon/Dimethylaminoethylmethacrylat-Copolymer |
| 0,20 g | Cetyltrimethylammoniumchlorid |
| 0,40 g | Polyvinylpyrrolidon/Vinylacetat-Copolymer |
| 0,20 g | Isopropanol |
| 97,22 g | Wasser |
| 100,00 g | |

94 g des vorstehenden Haarpflegemittels werden mit 6 g eines Gemisches aus 75 Gewichtsprozent n-Butan, 20 Gewichtsprozent i-Butan und 5 Gewichtsprozent Propan in einen geeigneten Aerosolbehälter abgefüllt. Das erfindungsgemäße Haarpflegemittel wird dem Behälter in Form eines Schaumes entnommen.

| **Beispiel 2:** | **Haarpflegeschaum** |
|---|---|
| 1,50 g | eines Gemisches aus 87 Gewichtsprozent cyclischem Dimethylpolysiloxan und 13 Gewichtsprozent alpha-Hydroxy,omega-hydroxypoly[oxy(dimethylsilylen)](Dow Corning® Fluid Q2-1401 der Firma Dow Corning GmbH, BRD) |
| 0,75 g | diquaternäres Polydimethylsiloxan (Abil®-Quat 3272 der Firma TH. Goldschmidt AG, BRD) |
| 0,10 g | Diallyldimethylammoniumchlorid/Acrylamid-Copolymer |
| 0,13 g | Cetyltrimethylammoniumchlorid |
| 3,00 g | Polyvinylpyrrolidon |
| 94,52 g | Wasser |
| 100,00 g | |

96 g des vorstehenden Haarpflegemittels werden mit 4 g eines Gemisches aus 50 Gewichtsprozent Propan, 40 Gewichtsprozent n-Butan und 10 Gewichtsprozent Dimethylether in einen Aerosolbehälter abgefüllt. Das erfindungsgemäße Haarpflegemittel wird beim Versprühen in Schaumform dem Behälter entnommen.

| **Beispiel 3:** | **Haartönungsschaum** |
|---|---|
| 1,0000 g | eines Gemisches aus 87 Gewichtsprozent cyclischem Dimethylpolysiloxan und 13 Gewichtsprozent alpha-Hydroxy,omega-hydroxypoly[oxy(dimethylsilylen)](Dow Corning® Fluid Q2-1401 der Firma Dow Corning GmbH, BRD) |
| 0,6000 g | diquaternäres Polydimethylsiloxan (Abil®-Quat 3272 der Firma TH. Goldschmidt AG, BRD) |
| 0,3000 g | Vinylimidazoliniummethochlorid/Vinylpyrrolidon-Copolymer |
| 0,2500 g | Cetyltrimethylammoniumchlorid |
| 0,7500 g | Vinylpyrrolidon/Vinylacetat-Copolymer |
| 0,0025 g | Basic Violet 14 (C.I.-Nr. 42 510) |
| 0,2500 g | Isopropanol |
| 9,4700 g | Ethanol |
| 87,3775 g | Wasser |
| 100,0000 g | |

92 g des vorstehenden Haartönungsmittels werden mit 8 g eines Gemisches aus 73 Gewichtsprozent n-Butan, 17 Gewichtsprozent Propan und 10 Gewichtsprozent i-Butan in einen Aerosolbehälter abgefüllt.

Beim Versprühen wird der erfindungsgemäße Haartönungsschaum erhalten.

| **Beispiel 4:** | **Haarpflegemittel in Form einer wäßrigalkoholischen Lösung** |
|---|---|
| 0,70 g | eines Gemisches aus 87 Gewichtsprozent cyclischem Dimethylpolysiloxan und 13 Gewichtsprozent alpha-Hydroxy, omegahydroxypoly[oxy(dimethylsilylen)](Dow Corning® Fluid Q2-1401 der Firma Dow Corning GmbH, BRD) |
| 1,00 g | diquaternäres Polydimethylsiloxan (Abil® Quat 3272 der Firma TH. Goldschmidt AG, BRD) |
| 0,16 g | Dimethyldiallylammoniumchlorid-Polymer |
| 0,13 g | Cetyltrimethylammoniumchlorid |
| 0,50 g | Polyvinylpyrrolidon |
| 11,36 g | Ethanol |
| 86,15 g | Wasser |
| 100,00 g | |

| **Beispiel 5:** | **Haarpflegemittel** |
|---|---|
| 1,400 g | eines Gemisches aus 87 Gewichtsprozent cyclischem Dimethylpolysiloxan und 13 Gewichtsprozent alpha-Hydroxy,omega-hydroxypoly[oxy(dimethylsilylen)](Dow Corning® Fluid Q2-1401 der Firma Dow Corning GmbH, BRD) |
| 0,800 g | diquaternäres Polydimethylsiloxan (Abil®-Quat 3272 der Firma TH. Goldschmidt AG, BRD) |
| 0,100 g | polymeres quaternäres Ammoniumsalz von Hydroxyethylcellulose (Polymer JR® der Firma Union Carbide, Schweiz) |
| 0,218 g | Cetyltrimethylammoniumchlorid |
| 0,500 g | Polyvinylpyrrolidon/Vinylacetat-Copolymer |
| 0,300 g | Polyvinylpyrrolidon |
| 96,682 g | Wasser |
| 100,000 g | |

| **Beispiel 6:** | **Haarpflegeschaum** |
|---|---|
| 0,90 g | eines Gemisches aus 87 Gewichtsprozent cyclischem Dimethylpolysiloxan und 13 Gewichtsprozent alpha-Hydroxy,omega-hydroxypoly[oxy(dimethylsilylen)](Dow Corning® Fluid Q2-1401 der Firma Dow Corning GmbH, BRD) |
| 0,25 g | diquaternäres Polydimethylsiloxan (Abil®-Quat 3272 der Firma TH. Goldschmidt AG, BRD) |
| 0,08 g | Vinylimidazoliniummethachlorid/Vinylpyrrolidon-Copolymer |
| 0,08 g | Dimethyldiallylammoniumchlorid |
| 0,28 g | Cetyltrimethylammoniumchlorid |
| 0,60 g | Polyvinylpyrrolidon/Vinylacetat-Copolymer |
| 97,81 g | Wasser |
| 100,00 g | |

90 g des vorstehenden Haarpflegemittels werden mit 10 g n-Butan in einen geeigneten Aerosolbehälter abgefüllt. Beim Versprühen wird das vorstehende erfindungsgemäße Haarpflegemittel dem Behälter als Schaum entnommen.

| **Beispiel 7:** | **Haarpflegeschaum** |
|---|---|
| 0,70 g | eines Gemisches aus 87 Gewichtsprozent cyclischem Dimethylpolysiloxan und 13 Gewichtsprozent alpha-Hydroxy,omega-hydroxypoly[oxy(dimethylsilylen)](Dow Corning® Fluid Q2-1401 der Firma Dow Corning GmbH, BRD) |
| 0,90 g | diquaternäres Polydimethysiloxan (Abil®-Quat 3272 der Firma TH. Goldschmidt AG, BRD) |
| 0,20 g | Vinylimidazoliniummethachlorid/Vinylpyrrolidon-Copolymer |
| 0,26 g | Cetyltrimethylammoniumchlorid |
| 0,48 g | Kokosfettsäureamidopropylbetain |
| 0,50 g | Polyvinylpyrrolidon/Vinylacetat-Copolymer |
| 96,96 g | Wasser |
| 100,00 g | |

Das vorstehende erfindungsgemäße Haarpflegemittel wird in einen flexiblen Behälter mit einem Aufsatz mit einer Schaumerzeugungseinrichtung gemäß der EP-B 0 460 154 abgefüllt. Durch mechanischen Druck kann dem Behälter der erfindungsgemäße Haarpflegeschaum entnommen werden.

| **Beispiel 8:** | **Haarpflegeschaum** |
|---|---|
| 0,80 g | eines Gemisches aus 87 Gewichtsprozent cyclischem Dimethylpolysiloxan und 13 Gewichtsprozent alpha-Hydroxy,omega-hydroxypoly[oxy(dimethylsilylen)](Dow Corning® Fluid Q2-1401 der Firma Dow Corning GmbH, BRD) |
| 1,20 g | diquaternäres Polydimethylsiloxan (Abil®-Quat 3272 der Firma TH. Goldschmidt AG, BRD) |
| 0,20 g | Dimethyldiallylammoniumchlorid-Polymer |
| 0,25 g | Cetyltrimethylammoniumchlorid |
| 0,12 g | Laurylpolyglucose |
| 0,60 g | Polyvinylpyrrolidon/Vinylacetat-Copolymer |
| 0,25 g | Isopropanol |
| 9,47 g | Ethanol |
| 87,11 g | Wasser |
| 100,00 g | |

94 g des vorstehenden erfindungsgemäßen Haarpflegemittels werden mit 6 g eines Gemisches aus 75 Gewichtsprozent n-Butan, 20 Gewichtsprozent i-Butan und 5 Gewichtsprozent Propan in einen geeigneten Aerosolbehälter abgefüllt. Das erfindungsgemäße Mittel wird dem Aerosolbehälter als Schaum entnommen.

| **Beispiel 9:** | **Haarpflegemittel** |
|---|---|
| 0,900 g | eines Gemisches aus 87 Gewichtsprozent cyclischem Dimethylpolysiloxan und 13 Gewichtsprozent alpha-Hydroxy,omega-hydroxypoly[oxy(dimethylsilylen)](Dow Corning® Fluid Q2-1401 der Firma Dow Corning GmbH, BRD) |
| 0,800 g | diquaternäres Polydimethylsiloxan (Abil®-Quat 3272 der Firma TH. Goldschmidt AG, BRD) |
| 0,100 g | Vinylpyrrolidon/Dimethylaminoethylmethacrylat-Copolymer |
| 0,174 g | Cetyltrimethylammoniumchlorid |
| 0,270 g | mit 45 Einheiten Ethylenoxid ethoxyliertes hydriertes Rizinusöl |
| 0,700 g | Polyvinylpyrrolidon/Vinylacetat-Copolymere |
| 97,056 g | Wasser |
| 100,000 g | |

Alle Prozentangaben in der vorliegenden Anmeldung stellen Gewichtsprozente dar.

## Patentansprüche

1. Mittel zur Pflege der Haare, das nicht ausgespült werden muß, auf wäßriger oder wäßrig-alkoholischer Basis, dadurch gekennzeichnet, daß es
(A) 0,5 bis 2,5 Gewichtsprozent eines Gemisches aus 87 Gewichtsprozent cyclischem Dimethylpolysiloxan der allgemeinen Formel (I) 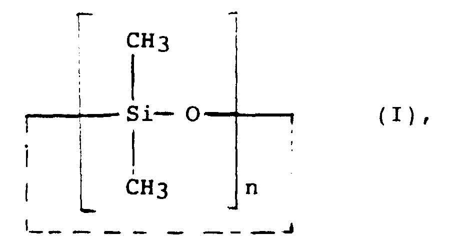 wobei n im Mittel 4 bis 6 bedeutet, und 13 Gewichtsprozent alpha-Hydroxy,omega-hydroxypoly[oxy(dimethylsilylen)],
(B) 0,25 bis 1,25 Gewichtsprozent eines diquaternären Polydimethylsiloxans,
(C) 0,10 bis 0,50 Gewichtsprozent mindestens eines kationischen Polymers, das kein Silikonpolymer ist,
(D) 0,05 bis 0,50 Gewichtsprozent mindestens eines kationischen Tensids und
(E) 0,10 bis 5,00 Gewichtsprozent Polyvinylpyrrolidon und/oder Polyvinylpyrrolidon/Vinylacetat-Copolymer
enthält und frei von anionischen Tensiden ist.

2. Mittel nach Anspruch 1, dadurch gekennzeichnet, daß es 0,50 bis 1,5 Gewichtsprozent der Komponente (A) enthält.

3. Mittel nach einem der Ansprüche 1 oder 2, dadurch gekennzeichnet, daß es 0,25 bis 0,75 Gewichtsprozent der Komponente (B) enthält.

4. Mittel nach einem der Ansprüche 1 bis 3, dadurch gekennzeichnet, daß es als Komponente (B) ein diquaternäres Polydimethylsiloxan der allgemeinen Formel (II) 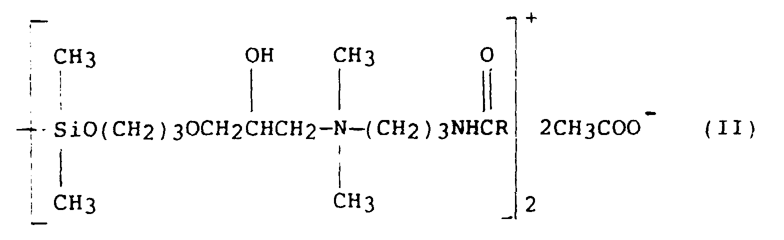 wobei R einen Kokosfettsäurealkylrest bedeutet, enthält.

5. Mittel nach einem der Ansprüche 1 bis 4, dadurch gekennzeichnet, daß es 0,10 bis 0,30 Gewichtsprozent der Komponente (C) enthält.

6. Mittel nach einem der Ansprüche 1 bis 5, dadurch gekennzeichnet, daß das kationische Polymer der Komponente (C) ausgewählt ist aus mit Dimethylsulfat quaternisiertem Vinylpyrrolidon/Dimethylaminoethylmethacrylat-Copolymeren, Diallyldimethylammoniumchlorid/Acrylamid-Copolymeren, Dimethyldiallylammoniumchlorid-Polymeren, polymeren quaternären Ammoniumsalzen von Hydroxyethylcellulose, Vinylimidazoliniummethachlorid/Vinylpyrrolidon-Copolymeren oder deren Gemisch.

7. Mittel nach einem der Ansprüche 1 bis 6, dadurch gekennzeichnet, daß es 0,10 bis 0,30 Gewichtsprozent der Komponente (D) enthält.

8. Mittel nach einem der Ansprüche 1 bis 7, dadurch gekennzeichnet, daß es als Komponente (D) Cetyltrimethylammoniumchlorid enthält.

9. Mittel nach einem der Ansprüche 1 bis 8, dadurch gekennzeichnet, daß es 0,2 bis 3,0 Gewichtsprozent der Komponente (E) enthält.

10. Mittel nach einem der Ansprüche 1 bis 9, dadurch gekennzeichnet, daß es zur gleichzeitigen Tönung des Haares 0,001 bis 2,0 Gewichtsprozent mindestens eines direkt auf das Haar aufziehenden Haarfarbstoffs enthält.

## Claims

1. Hair care agent that does not have to be rinsed out, on an aqueous or aqueous-alcoholic base, characterised in that it contains
(A) from 0.5 to 2.5 % by weight of a mixture of 87 % by weight of cyclic dimethylpolysiloxane of the general formula (I) 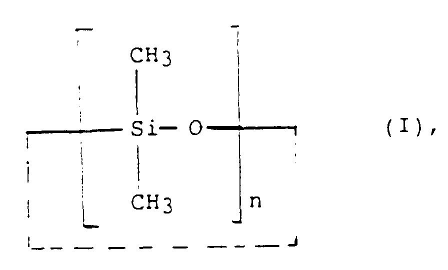 wherein n is on average from 4 to 6, and 13 % by weight of alpha-hydroxy,omega-hydroxypoly[oxy(dimethylsilylene)],
(B) from 0.25 to 1.25 % by weight of a diquaternary polydimethylsiloxane,
(C) from 0.10 to 0.50 % by weight of at least one cationic polymer that is not a silicone polymer,
(D) from 0.05 to 0.50 % by weight of at least one cationic surfactant and
(E) from 0.10 to 5.00 % by weight of polyvinylpyrrolidone and/or polyvinylpyrrolidone/ vinyl acetate copolymer
and is free of anionic surfactants.

2. Agent according to Claim 1, characterised in that it contains from 0.50 to 1.5 % by weight of component (A).

3. Agent according to either Claim 1 or Claim 2, characterised in that it contains from 0.25 to 0.75 % by weight of component (B).

4. Agent according to any one of Claims 1 to 3, characterised in that it contains as component (B) a diquaternary polydimethylsiloxane of the general formula (II) 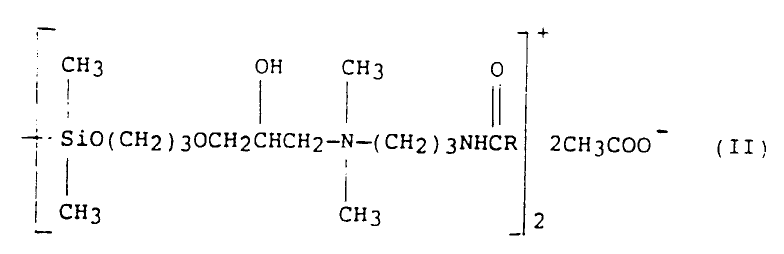 wherein R represents a coconut fatty acid alkyl residue.

5. Agent according to any one of Claims 1 to 4, characterised in that it contains from 0.10 to 0.30 % by weight of component (C).

6. Agent according to any one of Claims 1 to 5, characterised in that the cationic polymer of component (C) is selected from dimethyl sulphate-quaternised vinylpyrrolidone/dimethylaminoethyl methacrylate copolymers, diallyldimethylammonium chloride/acrylamide copolymers, dimethyldiallylammonium chloride polymers, polymeric quaternary ammonium salts of hydroxyethylcellulose, vinylimidazolinium methachloride/vinylpyrrolidone copolymers or a mixture thereof.

7. Agent according to any one of Claims 1 to 6, characterised in that it contains from 0.10 to 0.30 % by weight of component (D).

8. Agent according to any one of Claims 1 to 7, characterised in that it contains cetyltrimethylammonium chloride as component (D).

9. Agent according to any one of Claims 1 to 8, characterised in that it contains from 0.2 to 3.0 % by weight of component (E).

10. Agent according to any one of Claims 1 to 9, characterised in that, for the simultaneous tinting of the hair, it contains from 0.001 to 2.0 % by weight of at least one hair dye absorbed directly onto the hair.

## Revendications

1. Produit de soin des cheveux, ne devant pas être éliminé par rinçage, à base aqueuse ou alcoolo-aqueuse, caractérisé en ce qu'il contient
(A) de 0,5 à 2,5 pour-cent en poids d'un mélange constitué de 87 pour-cent en poids de déméthylpolysiloxane cyclique de formule générale (I) 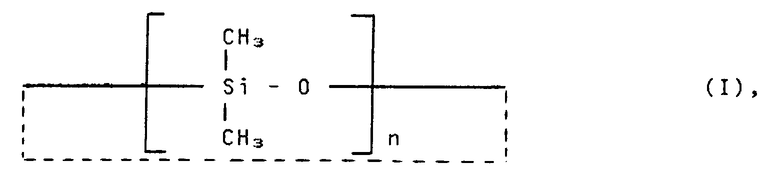 dans laquelle n vaut en moyenne de 4 à 6, et 13 pour-cent en poids de alpha-hydroxy, oméga-hydroxypoly[oxy(diméthyl-sylylène],
(B) 0,25 à 1,25 pour-cent en poids d'un polydiméthylsiloxane diquaternaire,
(C) 0,10 à 0,5 pour-cent en poids d'au moins un polymère cationique, qui n'est pas un polymère de silicone,
(D) de 0,05 à 0,5 pour-cent en poids d'au moins un agent tensioactif cationique et
(E) de 0,10 à 5,0 pour-cent en poids d'un polyvinylpyrrolidon et/ou d'un copolymère polyvinylpyrrolidon-acétate de vinyle,
et est exempt d'agents tensioactifs anioniques.

2. Produit selon la revendication 1, caractérisé en ce qu'il contient de 0,50 à 1,5 pour-cent en poids du composant (A).

3. Produit selon l'une des revendications 1 ou 2, caractérisé en ce qu'il contient de 0,25 à 0,75 pour-cent en poids du composant (B).

4. Produit selon l'une des revendications 1 à 3, caractérisé en ce qu'il contient comme composant (B) un polydiméthylsiloxane diquaternaire de formule générale (II) 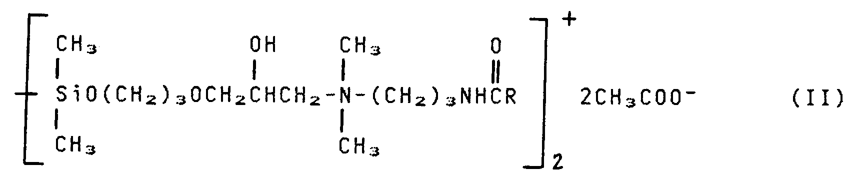 dans laquelle R est un résidu d'alkyle d'acide d'huile de coco.

5. Produit selon l'une des revendications 1 à 4, caractérisé en ce qu'il contient de 0,10 à 0,30 pour-cent en poids du composant (C).

6. Produit selon l'une des revendications 1 à 5, caractérisé en ce que le polymère cationique du composant (C) est choisi parmi le copolymère de vinylpyrrolidone diméthylaminoéthylméthacrylate quaternisé au sulfate de diméthyle, les copolymères de diallyldiméthylammonium-chlorure/acrylamide, les polymères de diméthyldiallyl-ammoniumchlorure, les polymères de sels d'ammonium quaternaires d'hydroxyéthylcellulose, les copolymères de vinylimidazoliniumméthachlorure/vinylpyrrolidone ou leur mélange.

7. Produit selon l'une des revendications 1 à 6, caractérisé en ce qu'il contient de 0,10 à 0,30 pour-cent en poids du composant (D).

8. Produit selon l'une des revendications 1 à 7, caractérisé en ce qu'il contient comme composant (D) du chlorure de cétyltriméthylammonium.

9. Produit selon l'une des revendications 1 à 8, caractérisé en ce qu'il contient de 0,2 à 3,0 pour-cent en poids du composant (E).

10. Produit selon l'une des revendications 1 à 9, caractérisé en ce qu'il contient pour assurer la coloration simultanée des cheveux de 0,001 à 2,0 pour-cent en poids d'au moins une teinture à cheveux à appliquer directement sur les cheveux.
